# EUROPEAN PATENT APPLICATION

(11) **EP 4 190 266 A1**
(43) Date of publication of application: **07.06.2023**
(21) Application number: 21212167.7
(22) Date of filing: 03.12.2021
(51) Int. Cl.: A61B 34/20

(54) **CAMERA-BASED TRACKING SYSTEM**

(71) Applicant: Koninklijke Philips N.V., 5656 AG Eindhoven (NL)
(72) Inventor: SPLIETHOFF, Jarich Willem, Eindhoven (NL); HENDRIKS, Bernardus Hendrikus Wilhelmus, Eindhoven (NL); GARCIA TORMO, Albert, Eindhoven (NL); MUELLER, Manfred, Eindhoven (NL)
(74) Representative: Philips Intellectual Property & Standards

(57) **Abstract**

The present invention relates to operating a camera-based tracking system. In order to provide accurate tracking at an earlier stage, a control device (10) for operation of a camera-based tracking system is provided. The control device comprises a controller (12) and a control signal output (14). The controller is configured to generate at least one operation signal for the tracking system that comprises, at least during a starting phase of the tracking system, at least one calibration compensation instruction. The at least one calibration compensation instruction comprises instructions for at least one of the group of: i) actively adjusting a temperature of the camera-based tracking system, and ii) adapting a calibration-related camera output of the camera-based tracking system, which camera output is used for a tracking calculation. The control signal output is configured to provide the at least one operation signal to the tracking system. In one approach, a pre-heating is provided in order to achieve (steady) calibration state by providing a control signals to the cameras for operation in a high thermal energy production mode. In another approach, the calibration is provided as dynamic calibration model that allows a calibration also for states of the system outside the steady state.

## Description

### FIELD OF THE INVENTION

The present invention relates to a control device for operation of a camera-based tracking system, to a camera-based tracking system and to a method for operating a camera-based tracking system.

### BACKGROUND OF THE INVENTION

Navigation systems are used in medical interventions for tracking instruments. For example, instruments are provided with markers that are detected by a tracking system. One example for tracking markers is the use of optical tracking systems where cameras track markers in the image data provided by the cameras. As an example, the position of a tracked instrument in relation to the patient's anatomy is shown on images of the patient, as the surgeon moves the instrument. The surgeon thus uses the system to 'navigate' the location of an instrument. The feedback of the instrument location provided by the system may be useful in situations where the surgeon cannot actually see the tip of the instrument, such as in minimally invasive surgeries. Tracking devices is also provided in computer-aided surgery (CAS) systems which CAS systems are increasingly used for preoperative planning and also for navigation of instruments during surgical procedure. As an example, using the surgical navigation system, the surgeon uses special instruments, which are tracked by the navigation system. Tracking or navigation systems may be moved between operating rooms. Clinical uses and schedules may demand that the systems should be easy to setup, boot-up quickly and be ready for use within a few minutes. However, it has been shown that optical tracking systems used for surgical navigation may take up to half an hour or even one hour to warm-up and stabilize after a cold-start. Due to this start-up behavior, the system's ability to track markers attached to the patient and surgical instruments is temporarily impaired and results might be inaccurate.

### SUMMARY OF THE INVENTION

There may thus be a need for improved accurate tracking, e.g. at an earlier stage.

The object of the present invention is solved by the subject-matter of the independent claims; further embodiments are incorporated in the dependent claims. It should be noted that the following described aspects of the invention apply also for the device for operation of a camera-based tracking system, for the camera-based tracking system and for the method for operating a camera-based tracking system.

According to the present invention, a control device for operation of a camera-based tracking system is provided. The control device comprises a controller and a control signal output. The controller is configured to generate at least one operation signal for the tracking system that comprises, at least during a starting phase of the tracking system, at least one calibration compensation instruction. The at least one calibration compensation instruction comprises instructions for at least one of the group of: i) actively adjusting a temperature of the camera-based tracking system, and ii) adapting a calibration-related camera output of the camera-based tracking system, which camera output is used for a tracking calculation. The control signal output is configured to provide the at least one operation signal to the tracking system.

As an effect, the compensation instruction is provided in order to at least partly compensate a divergence between a current condition of the camera-based tracking system and a predetermined condition of the system assumed for a calibration of the system. This improves accuracy of the tracking system.

According to an example, the calibration compensation instruction comprises at least one control signal for temporary operating the camera-based tracking system in an increased heat generating mode.

According to an example, for temporary operating the camera-based tracking system in the increased heat generating mode, the calibration compensation instruction comprises at least one control signal for temporary operating at least one of the at least two cameras in a heat-up mode to generate heat by the at least one of the at least two cameras. The at least one control signal is configured to operate the at least one of the at least two cameras in an increased energy consumption mode where a higher energy consumption by the camera takes place than in a regular tracking mode when the camera is operated for tracking. The control signal output is configured to provide the at least one operation signal to the tracking system in order to operate the at least one of the at least two cameras in the increased energy consumption mode and to thereby heat up at least the camera due to a higher energy consumption as compared to the regular tracking mode.

According to an example, for the increased heat generating mode, the calibration compensation instruction comprises at least one control signal for temporary operating a heating element of the camera-based tracking system.

In an example, markers are used for tracking. In another example, marker-less tracking is provided.

This addresses that, for an optical tracking system, it is desirable to decrease warm-up time and minimize the system's temporary decreased accuracy at during a cold-start. A further advantage is that cameras can be configured from an external computer. Still further, this is based on that power consumption of the cameras is directly related to heat generation by the cameras and subsequent dissipation of this heat to the parts of the system that are thermally connected to the cameras (heat sink). More heat generation in the cameras leads to a steeper increase in temperature of the cameras and thermally connected components. It is noted that that much of the thermal stabilization process happens inside the camera. For example, the camera housing expands, the position of the sensor moves, etc., which is addressed by the present solution without the need for mayor changes to the camera. The warm-up of the cameras, to a level that is needed for stable operation of the system, can further be accelerated by using a set of camera parameters that demand more power consumption than needed for actual normal operation.

Tracking of markers, e.g. on patients and devices or instruments, is achieved by determining their exact spatial location in relation to a tracking system. The knowledge of the spatial location of the tracking system within a local reference grid, or spatial reference system, within the operation room, allows to link and embed the markers into the local reference grid. The determination of the spatial location of the markers is achieved basically by trigonometry. For example, distances to a marker are measured from different sources, i.e. from different cameras. By trigonometric calculations, the spatial location in relation to the cameras can be determined. The trigonometric calculations allow to spatially register images provided by the cameras. However, this requires a precise knowledge of the actual (spatial) location of the cameras and also their (spatial) orientation. This precise geometric knowledge is provided by a calibration of the camera system. To implement a camera system, several cameras are provided, which cameras must be fixed to a basis. For this reason, the cameras are physically mounted to a common support structure. Since the support structure is always subject to thermal expansion, depending on material, design and size of course, there will always be geometrical changes that affect the position and orientation of the cameras, which then influences their preciseness of detecting the markers. The thermal expansion is mainly given by the internal heat generated by the operation of the cameras. The heat provides a thermal expansion of the cameras themselves (optical system in relation to the image detector element), of the mounts, with which the cameras are fixed to the support structure, and of the support structure itself. Consequently, a calibration of a system is provided that considers a state which is assumed to be stable. Such state is taken for conditions during normal operation of the tracking system, i.e. a situation during use of the system.

The present invention addresses this by measures to provide conformity of the current state and the assumed state for the calibration. By providing additional heat, the system's state is better matching the assumed state applied for calibration.

According to an example, the controller is configured to take measured external conditions into account for the control signal when generating the operation signal. The external conditions comprise at least one of the group of: ambient temperature, illumination conditions and indoor air currents.

According to an example, the controller is configured to provide a dynamic calibration model for the camera-based tracking system. The dynamic calibration model comprises a plurality of calibration factors depending on a temperature related state of the system. For a current temperature related state of the camera-based tracking system, the controller is also configured to determine a matching calibration factor from the plurality of calibration factors. Further, the control signal output is configured to provide the matching calibration factor for application during operation of the camera-based tracking system.

In an example, the controller is configured to provide the calibration compensation instruction comprising the operation instructions to operate the at least one of the at least two cameras in the increased energy consumption mode. The controller is also configured to provide the dynamic calibration model for the camera-based tracking system and to determine and provide the matching calibration factor. Further, the controller is further configured to operate the at least two cameras in an imaging mode while operating the cameras in the increased energy consumption mode. The controller is further configured to apply the matching calibration factor to generated images.

According to the present invention, also a camera-based tracking system is provided. The system comprises a plurality of at least two cameras and a control device according to one of the preceding examples. The at least one operation signal is provided for operating at least one of the at least two cameras.

In an option, the camera-based tracking system is a camera-based medical tracking system for use in a hospital or other healthcare related setting.

According to an example, the increased energy consumption mode comprises an image acquisition with an energy consumption that is at least 50% higher than an energy consumption during a normal tracking mode of the system.

In an option, the increased energy consumption mode comprises an image acquisition with at least one of the group of the following being at least 50% increase compared to a normal tracking mode of the system: frame rate, exposure time, bit depths, bit packing, area of interest, clocking frequency, color formats, image compression and on-board image pre-processing.

According to an example, a set of markers is provided that can be tracked by the system. The markers are attachable to at least one of the group of subjects, objects and devices.

In another example, marker-less tracking is provided.

According to the present invention, also a method for operating a camera-based tracking system is provided. The method comprises the following steps:
- Generating at least one operation signal for the tracking system that comprises, at least during a starting phase of the tracking system, at least one calibration compensation instruction. The at least one calibration compensation instruction comprises instructions for at least one of the group of: i) actively adjusting a temperature of the camera-based tracking system, and ii) adapting a calibration-related camera output of the camera-based tracking system, which camera output is used for a tracking calculation.
- Providing the at least one operation signal to the tracking system.
- Operating the camera-based tracking system based on the operation signal comprising at least one calibration compensation instruction.

According to an aspect, tracking errors due to a not yet prevailing calibration status are minimized by providing counter measures to compensate for tracking errors.

According to one approach, a pre-heating is provided in order to achieve the (steady) calibration state (applied for calibration of the system) by providing a control signals to the cameras for operation in a high thermal energy production mode. Existing equipment is thus used for generating heat. The thermal energy is thus provided exactly where it has the largest effect, namely at the camera positions. The pre-heating is mimicking the heat generated during normal operation, but in a faster, boosted mode for example.

According to another approach, instead of trying to achieve a steady state which forms the basis for a static calibration by pre-heating, the calibration is provided as dynamic calibration model that allows a calibration also for states of the system outside the steady state. Even when operation temperature is not reached yet, a respectively adapted calibration is provided by the dynamic model.

According to a further approach, combining the two approaches above, the dynamic model is provided in combination with the heating-up procedure by special operation signals for the cameras. In combination with optional temperature sensors, the accuracy can be further improved.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a control device for operation of a camera-based tracking system.
Fig. 2 shows an example of a camera-based tracking system in the context of a cath lab.
Fig. 3a shows an example of a camera-based tracking system within a housing structure.
Fig. 3b shows the example of the camera-based tracking system of Fig. 3a without the housing structure.
Fig. 4 shows steps of an example of a method for operating a camera-based tracking system.
Fig. 5 shows a graph indicating tracking errors in relation to time after start.
Fig. 6 shows a graph indicating temperature of the system in relation to time after start.
Fig. 7 shows a graph indicating system temperature over time for different operating modes.
Fig. 8 shows a graph indicating an example of a warm-up pattern with system temperature and camera power consumption over time.
Fig. 9 shows a graph indicating another example of a warm-up pattern with system temperature and camera power consumption over time.
Fig. 10 shows a graph indicating a further example of a warm-up pattern with system temperature and camera power consumption over time.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

Surgical navigation systems that rely on optical cameras may have an intrinsic accuracy for marker-tracking in the range 0.5 - 1 mm. The intrinsic 3D marker tracking accuracy of the system is optimal (smallest) when the system is in the same "state" as when the system was calibrated. If the system is in a different state, this will lead to less accurate marker tracking, thus a larger 3D error. An example is that the relative geometric position and/or orientation of the optical cameras has changed due to mechanical forces (shocks, vibrations). This may require recalibration of the system. Furthermore, when using an optical camera system, temperature is a significant factor that might introduce changes in the actual image that lead to additional tracking errors in the same order of magnitude of the desired accuracy. As an example, during start-up of the navigation system, tracking accuracy can be temporary decreased as the hardware is in a "different state" due to temperature-related effects. There are two main effects that cause measurable thermally-induced error: First, the frame that suspends the optical camera will, to a certain extent, be subjected to temperature-related expansion, causing a temporary different geometric relation between the cameras. Second, thermally-related changes of the camera housing and lens system cause measurable differences in projection of the image on the camera imaging sensor. Both effects contribute to a temporary decrease in accuracy, as the calibration is performed when the system is in thermal steady state and state and during the transient the system is (temporarily) different.

Roughly, the start-up of an optical tracking system can be divided into two stages. In a first stage, also referred to as stage 1, a warm-up of the system takes place: At the start, the temperature of the camera is close to ambient temperature of the system. It will increase due to heat-dissipation of the active components, e.g. camera processor, imaging chip etc. The temperature-related tracking error is maximal at the start of this phase and minimal at the end of this phase. In a second stage, also referred to as stage 2, a steady-state of the system takes is reached: The hardware reached a thermal equilibrium and optical tracking accuracy is at the same level as in the calibration state.

In an example, the thermal behavior of the optical tracking system might be more complex, as there may be a certain delay in heat transfer from the camera to the other parts of the system that are thermally connected and serve as heat-sink. Still, preferably the warm-up behavior of a navigation system is such that the thermal response is minimal and that the time needed to reach steady-state after start-up is minimal. The present solution does not necessarily need smart hardware choices and/or design of the system, thus avoiding further constructional related costs. Another aspect for causing calibration affecting changes can be provided by differences in ambient temperatures e.g. by moving the camera from a hot storage room to an air-conditioned operation room.

If an optical tracking system is warming up from the ambient temperature of its surroundings, its internal components inevitably change dimension and shape, resulting in start-up behavior. The present solution speeds-up the warm-up of the optical tracking system to a steady-state level and decreases the temperature-related difference in tracking accuracy such that tracking can be performed with an optimal level of accuracy soon(er) after start-up without adding hardware and/or hardware complexity to the system.

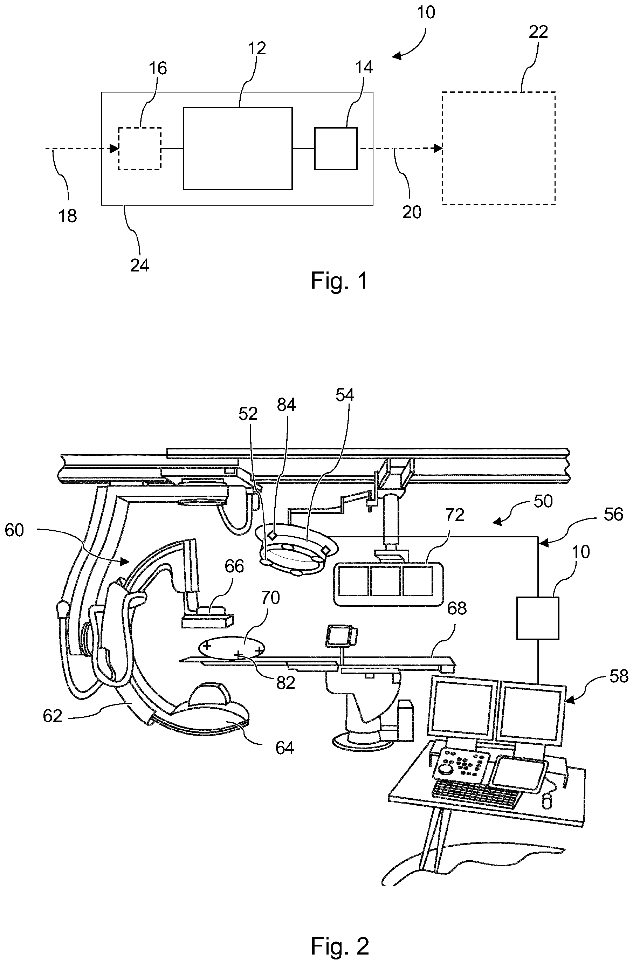

Fig. 1 schematically shows an example of a control device 10 for operation of a camera-based tracking system. The control device comprises a controller 12 and a control signal output 14. The controller 12 is configured to generate at least one operation signal for the tracking system that comprises, at least during a starting phase of the tracking system, at least one calibration compensation instruction. The at least one calibration compensation instruction comprises instructions for at least one of the group of: i) actively adjusting a temperature of the camera-based tracking system, and ii) adapting a calibration-related camera output of the camera-based tracking system, which camera output is used for a tracking calculation. The control signal output 14 is configured to provide the at least one operation signal to the tracking system in order to operate the camera-based tracking system.

In an option, shown in Fig. 1 by hashed lines, a signal input 16 is provided. The signal input 16 is configured to receive a signal for activating a camera-based tracking system comprising at least two cameras. Based on the activating signal, the controller is configured to generate the at least one operation signal.

Hence, the at least one calibration compensation instruction is provided upon starting the system and allows a faster use of the system.

In another option, the signal input 16 is configured to receive a signal for ongoing operation of the camera-based tracking system. Based on the ongoing operation signal, the controller is configured to generate the at least one operation signal as a check of the calibration.

Hence, the at least one calibration compensation instruction is provided during use of the system upon starting the system and allows an improved accuracy during operation with e.g. changing environmental conditions.

A first arrow 18 indicates the signal for activating the camera-based tracking system. A second arrow 20 indicates the output of the at least one operation signal, for example to a camera-based tracking system, indicated with dotted frame 22. A further frame 24 indicates that the controller 12, the control signal output 14 and the signal input 16 can be provided in a common structure like a housing. However, they can also be arranged separately, but data-connected.

The compensation instruction is provided in order to at least partly compensate a divergence between a current condition of the camera-based tracking system and a predetermined condition of the system assumed for a calibration of the system.

The actively adjusting of the temperature of the camera-based tracking system is provided in order to at least partly compensate the divergence.

The adapting of the camera output is provided in order to at least partly compensate the divergence.

The control signal output 14 is configured to provide the at least one operation signal to the tracking system in order to operate the camera-based tracking system based on the operation signal comprising at least one calibration compensation instruction and thereby reducing the divergence between the current condition of the camera-based tracking system and the predetermined condition of the system assumed for the calibration of the system.

The calibration compensation instruction can also be referred to as calibration modification instruction. The instruction is provided in order to adjust or modify the system's settings regarding the calibration.

The calibration compensation instruction provides to minimize the offset between the assumed state on which calibration is based and the current state of the system.

As an effect, the system is provided with improved accuracy even in situations when the static calibration state is not yet achieved, like during starting phases.

The starting phase refers to a part of the operation from starting the operation of the system until a predetermined condition of the system assumed for a calibration of the system is reached.

In a first option, not further shown in detail in Fig. 1, the calibration compensation instruction comprises at least one control signal for temporary operating the camera-based tracking system in an increased heat generating mode.

In an option, also not further shown in detail in Fig. 1, for temporary operating the camera-based tracking system in the increased heat generating mode, the calibration compensation instruction comprises at least one control signal for temporary operating at least one of the at least two cameras in a heat-up mode to generate heat by the at least one of the at least two cameras. The at least one control signal is configured to operate the at least one of the at least two cameras in an increased energy consumption mode where a higher energy consumption by the camera takes place than in a regular tracking mode when the camera is operated to track markers. The control signal output 14 is configured to provide the at least one operation signal to the tracking system in order to operate the at least one of the at least two cameras in the increased energy consumption mode and to thereby heat up at least the camera due to a higher energy consumption as compared to the regular tracking mode.

The divergence can also be referred to as discrepancy.

In one option, the heating-up mode is provided by operating the (already existing) cameras in a heat-up mode for generating the thermal energy.

In another option, the heating-up mode is provided by operating separate heating elements for generating the thermal energy. The separate heating elements are provided with the tracking systems. The heating elements can be provided by separately attached heating elements or by heating elements integrally formed with the cameras, with mounts for the cameras or a with a support structure. The heating elements can also be provided by identifying operating elements like circuits in the system which can be operated in a designated thermal energy generation mode.

In an example, for the increased heat generating mode, the calibration compensation instruction comprises at least one control signal for temporary operating a heating element of the camera-based tracking system.

In an example, the heating element comprises at least one integrated heating element. For example, the heating element is provided in a camera mount, such that the heating element provides thermal energy at a location where a camera in its operating mode transfers thermal energy to a support structure.

In another example, the integrated heating element is provided within a camera next to a location of an electric circuit that generates heat when the camera is in its operating mode.

An example for such additional heating elements are resistor stripes attached to existing structures. A targeted heat generation (and preheating or warming-up) is thus possible with less energy consumption compared to e.g. heating up the complete arrangement of the system in a warming-up area of an operation room or even in a separated warming-up chamber.

In an example, the higher energy consumption is provided by an increased heat generation due to the operation. For example, an electric component, e.g. a certain part of an electric circuit, is activated that uses same energy as other parts during normal operation, but which part or component produces more temperature, i.e. more heat available for dissipation and thus heating up of the system.

As an effect, the heat (to reach the operation conditions upon which calibration is based) is generated exactly at the location where it should be generated. As an advantage in terms of economy and reduced system complexity, this is achieved by using already available hardware. Another advantage results in that the tracking system's functionality is maintained. Even though at the start of the heat-up process, the tracking may be less accurate, the system is available for other purposes for which that might not be such an issue; like already providing visualization of the region of interest to allow a surgeon to position an interventional device in a convenient location while still maintaining adequate line of sight or the like.

The term "activating" relates to the beginning of an operation of the tracking system when the system is started, e.g. converted or transitioned, from an OFF-mode to an ON-mode. This is also referred to as starting. The activating or starting also comprises to transition the system from a stand-by-mode or sleeping mode to an ON-mode. The ON-mode is a mode in which the system is ready for use, i.e. ready for the normal designated operation. The ON-mode can also be referred to as operation-mode. It is noted that the term operation relates to the system in this context and does not necessarily mean that a (medical) operation takes place.

The "increased energy consumption mode" relates to a mode that results in an energy consumption of the camera(s) that is higher than without the operational signal for the increased energy consumption mode. The increased energy consumption mode can also be referred to as higher energy consumption mode. The energy consumption can also be referred to as the use of (electric) power.

The "increased energy consumption mode" can also be referred to as artificial or triggered warm-up mode, or as boosted warming-up mode.

As an effect, due to the operation in the high energy use mode, the warm-up period is reduced.

Within the present context, the term "normal operation" relates to the system settings as applied when making regular images with the cameras, e.g. for tracking purposes.

The term "normal operation" relates to requirements, such as for example, camera frame rate, i.e. images per second. As an example, to make surgical navigation work "smooth", according to current requirements, the cameras are adjusted to provide at least twelve frames per second (fps).

In an example, the term "increased energy consumption mode" relates to cameras forced to work "harder", e.g. thirty frames per second during the warm-up. This reduces warm-up time significantly.

In an example, an optical tracking system is provided that is configured such that during start-up, the optical cameras in the systems are configured to consume more power than during normal operation. This is achieved by setting the camera parameters, e.g. frame rate, acquisition time, color formats, image compression, on-board image pre-processing, clocking of the camera, to a value/state that requires more on-board image processing and therefore more power consumption. More power consumption leads to more heat dissipation, which leads to a quicker warm-up of the camera and thermally connected components than in case of lower power-consumption settings. Once a temperature is reached that corresponds to the temperature that would normally be reached with lower power consumption settings, the camera settings are adjusted accordingly. As a result, less time is needed to bring the optical system at operating temperature.

In an example, not further shown in detail in Fig. 1, a control device for operation of a camera-based tracking system is provided. The control device comprises a signal input, a controller and a control signal output. The signal input is configured to receive a signal for activating a camera-based tracking system comprising at least two cameras. Based on the activating signal, the controller is configured to generate at least one operation signal for the tracking system that comprises at least one control signal for temporary operating at least one of the at least two cameras in a heat-up mode to generate heat by the at least one of the at least two cameras. The at least one control signal is configured to operate the at least one of the at least two cameras in an increased energy consumption mode where a higher energy consumption by the camera takes place than in a regular tracking mode when the camera is operated to track markers. The control signal output is configured to provide the at least one operation signal to the tracking system in order to operate the at least one of the at least two cameras in the increased energy consumption mode and to thereby heat up at least the camera due to a higher energy consumption as compared to the regular tracking mode.

In an example, the controller is configured to provide an operation signal to operate the at least one camera in the increased energy consumption mode until a predetermined thermal condition is reached.

In an option, the controller is configured to provide an operation signal to maintain the increased energy consumption mode until at least one of the group of the at least one camera and the system has reached its normal operation temperature which is the basis for the system's calibration.

In another option, the controller is configured to provide an operation signal to provide the increased energy consumption mode beyond the point when the at least one camera has reached its normal operation temperature.

In an example, the controller is configured to provide a normal operation signal to operate the at least one camera under normal conditions, after the heating up of the at least one camera by operation of the at least one of the at least two cameras in the increased energy consumption mode.

In an option, the controller is configured to modify the settings for normal operation based on the difference between the system temperature at time of calibration and the expected steady state temperature of the system under real-time circumstances.

In an example, not further shown in Fig. 1, the controller is configured to take measured external conditions into account for the control signal when generating the operation signal. The external conditions comprise at least one of the group of: ambient temperature, illumination conditions and indoor air currents.

In an example, external temperature conditions are measured and taken into account. For example, when ambient temperature is high, also the thermal equilibrium temperature of the optical system will be higher. In that case, the threshold temperature for switching from high-power to lower power consumption should be adjusted accordingly, e.g. earlier.

In an example, illumination conditions are measured and taken into account. Brightness of the illumination can cause higher (2-3 C°) steady-state camera temperatures the brightness can directly be deducted from the images recorded with the camera and therefore serve as additional input; likewise, if the external illumination cannot be controlled, the brightness in the captured image can still be adjusted via camera parameters (e.g. exposure time).

In an example, indoor air currents are considered, also referred to as air streams, provided for supply of clean air or for cooling or heating purposes, are either detected or provided as known parameters, as air movement will also affect the cameras due to the convecting effect.

In an example, the signal input 16 is provided configured to provide the measured external conditions.

In an example, also not further shown in Fig. 1, for generating the at least one operation signal for the tracking system, the controller is configured to provide a model of the warm-up of the system and its dynamic thermal behavior and to take these into account for the at least one control signal. The at least one control signal comprises a signal sequence comprising a plurality of varying control sub-signals.

In an example, the dynamic thermal behavior of the system is taken into account by using a model of the warm-up of the system. Rather than just switching from high-power consumption to lower-power consumption settings based on a threshold temperature, a priori knowledge of the system thermal behavior can be used to fine-tune the exact moment that camera parameters that affect power consumptions are changed during the warm-up.

In another option, also not further shown in detail in Fig. 1, the controller 12 is configured to provide a dynamic calibration model for the camera-based tracking system. The dynamic calibration model comprises a plurality of calibration factors depending on the temperature related state of the system. For a current temperature related state of the camera-based tracking system, the controller 12 is configured to determine a matching calibration factor from the plurality of calibration factors. The control signal output 14 is configured to provide the matching calibration factor for application during operation of the camera-based tracking system.

In a basic version of this option, the current temperature related state of the camera-based tracking system is an information about an activation of the system switching from an OFF state to an ON state. In another version, an information is provided on a time period since when the system is activated. In a further version, one or more temperature measurements, e.g. temperature readings from internal and/or external sensors are provided.

In a further option, the controller 12 is configured to provide the calibration compensation instruction comprising the operation instructions to operate the at least one of the at least two cameras in the increased energy consumption mode. The controller 12 is also configured to provide the dynamic calibration model for the camera-based tracking system and to determine and provide the matching calibration factor. The controller 12 is further configured to operate the at least two cameras in an imaging mode while operating the cameras in the increased energy consumption mode. The controller 12 is configured to apply the matching calibration factor to generated images.

In an example, the signal input 16 is provided configured to provide a current temperature related state of the camera-based tracking system. The controller is configured to provide the dynamic calibration model for the camera-based tracking system. The controller is also configured to determine a matching calibration factor from the plurality of calibration factors. The control signal output is configured to provide the matching calibration factor for application during operation of the camera-based tracking system.

In an example, also referring to the first and second option above, the controller 12 is configured to provide the calibration compensation instruction comprising the operation instructions to operate the at least one of the at least two cameras in the increased energy consumption mode. The controller 12 also is configured to provide the dynamic calibration model for the camera-based tracking system and to determine and provide the matching calibration factor. The controller 12 is further configured to operate the at least two cameras in an imaging mode while operating the cameras in the increased energy consumption mode. The controller 12 is furthermore configured to apply the matching calibration factor to generated images.

Fig. 2 shows an example of a camera-based tracking system 50 in the context of a cath lab. The camera-based tracking system 50 comprises a plurality of at least two cameras 52. An example of the control device 10 according to one of the preceding and following examples is provided. The at least one operation signal is provided for operating at least one of the at least two cameras.

In an option, the camera-based tracking system 50 is a camera-based medical tracking system for use in a hospital or other healthcare related setting.

In another option, the camera-based tracking system 50 is a camera-based tracking system for use in a non-medical setting.

In an example, a medical optical tracking system is provided that comprises a camera-based system capable of imaging medical instruments/objects and that also comprises means to measure the transient warm-up period, directly or indirectly.

Fig. 2 shows four of the cameras 52 mounted to a ring-like support structure 54. A schematically shown data connection 56 connects the four cameras and the control device 10. For example, the control device 10 is connected to a console 58 provided for operation of the different equipment in the cath lab. As an example, an X-ray imaging system 60 is provided with a C-arm 62 having an X-ray source 64 and an X-ray detector 66 mounted to opposite ends of the C-arm 62. The C-arm 62 is movably mounted to a ceiling rail structure. Further, a subject support 68, also referred to as patient table, for receiving a subject 70 is provided. Further, a display arrangement 72 is shown.

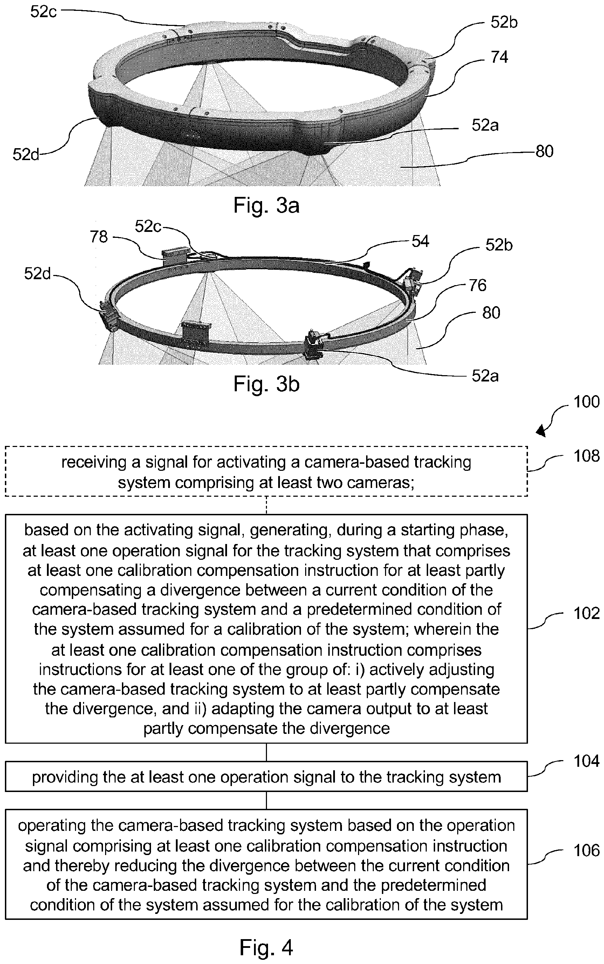

Fig. 3a shows an example of the camera-based tracking system 50 within a housing structure 74 with four cameras 52a, 52b, 52c and 52d. It is noted that also less cameras, for example two or three, or more cameras, for example five, six, seven, eight, nine or ten or more cameras can be provided.

Fig. 3b shows the example of Fig. 3a without the housing structure 74. A ring support 76 is shown to which the four cameras 52a, 52b, 52c and 52d are mounted. Further, connecting flanges 78 are also provided. Lines 80 below the cameras indicate their overlapping fields of vision.

In an example, the increased energy consumption mode comprises an image acquisition with an energy consumption that is at least 50% higher than an energy consumption during a normal tracking mode of the system. As an option, it is provided that the increased energy consumption mode comprises an image acquisition with at least one of the group of the following being at least 50% increased, compared to a normal tracking mode of the system: frame rate, exposure time, bit depths, bit packing, area of interest, clocking frequency, color formats, image compression and on-board image pre-processing.

The exposure time can also be referred to as acquisition time.

The image compression may also relate to debayering.

As an example, the increased frame rate is at least 50% higher than a frame rate used for a normal tracking mode of the system.

In an example, the increased frame is 1½-fold of the normal tracking frame rate.

In an example, the increased energy consumption mode comprises an image acquisition with an energy consumption that is at least the 2½ fold of an energy consumption during a normal tracking mode of the system.

In an example, the increased frame rate is provided as the double or more of the normal tracking frame rate, such as the 2½-fold of the normal tracking frame rate.

In an example, the at least one of the group of exposure time, bit depths, bit packing, area of interest, clocking frequency, color formats, image compression and on-board image pre-processing is provided in the double or 2½-fold amount.

In an example of the device or system, the control signal is configured such that the at least one camera is operated in the increased energy consumption mode until a predetermined thermal condition is reached.

As an option, the control signal is configured such that the increased energy consumption mode is maintained until at least one of the group of the at least one camera and the system has reached its normal operation temperature which is the basis for the system's calibration.

As a further option, the control signal is configured such that the increased energy consumption mode is provided beyond the point when the at least one camera has reached its normal operation temperature.

In an example of the device or system, the control signal is configured such that, after heating up the at least one camera by operating the at least one of the at least two cameras in the increased energy consumption mode, a normal operation signal is provided and the at least one camera is operated under normal conditions.

As an option, the control signal is configured such that the settings for normal operation are modified based on the difference between the system temperature at time of calibration and the expected steady state temperature of the system under real-time circumstances.

In an example of the device or system, the controller, for generating the at least one operation signal for the tracking system, is configured to provide a model of the warm-up of the system and its dynamic thermal behavior and to take these into account for the at least one control signal. As an option it is provided that the at least one control signal comprises a signal sequence comprising a plurality of varying control sub-signals.

In an example, at least one temperature sensor is provided to monitor the camera temperature. The camera temperature is taken into account for the generation of the operation signal. As an option, the controller is configured to generate an updated operation signal.

In an example, an internal built-in temperature sensor of the camera is used to monitor the camera temperature. The sensor can be read-out using the existing computer-camera communication interface without the need of an additional temperature measurement system.

In an example, not further shown, the controller 12 is configured to modify the operation signal based on at least one of the group of sensor data and system status.

For example, an internal temperature sensor measures the system temperature and then the controller decides on whether additional heating is beneficial.

For example, if the system has only been disconnected for a few minutes to move it from one operation room to the next and it is still near the steady state temperature assumed for calibration, a further additional heating may not be required.

For example, if the system was stored in an un-airconditioned storage room during a heat wave, a further additional heating may not be required.

In another option, temperature sensors provide temperature readings throughout the normal operation of the system and the controller compares the readings with a threshold. When falling below the threshold, the controller provides an activation of an intermediate warming-up procedure in which at least one of the at least two cameras is operated in the increased energy consumption mode thereby heating up at the camera due to a higher energy consumption as compared to the regular tracking mode. This ensures that the system is operating in those conditions that are the basis for the calibration.

In an example, not further shown, a plurality of sensors 84 (see Fig. 2) is provided that monitor a thermal behavior of the system. The thermal behavior as monitored by the sensors is used for providing a more accurate model of a warm-up of the system.

In an option, it is provided that the model of the warm-up of the system is used in combination with a plurality of temperature readings from sensors during warm-up to dynamically adjust the calibration of the system.

In an option, it is provided that a temperature history of the system is provided, and a remaining time is predicted before the system reaches a normal operation stage, and, as an option, the remaining time is indicated.

In an example, the model of the thermal behavior of the system in combination with temperature readings during warm-up and normal operation are used to dynamically adjust the optical calibration of the system. In an example, an optical calibration look-up table is created by calculating the optical calibration (internal and external camera parameters) at various points in time during warm-up of the system under various ambient-temperature conditions. In an option, this look-up table is subsequently used to dynamically adjust the optical calibration of the system based on the thermal state of the system.

In an example, a multitude of temperature sensors is used at various places on the optical tracking system such that a more accurate model can be applied for the system's dynamic thermal behavior. Within the cameras, the temperatures will change most drastically (typically ambient temperature up to 70 C°) and rapidly (as the heat is generated within the camera), whereas the camera housing already shows significant different temperature range (ambient temperature up to 50 C°) and a delayed warm-up. The warm-up of other components of the optical tracking system that are thermally connected to the cameras (e.g. camera mounts, frame) will lag behind and thermal equilibrium temperatures will be lower if no other sources of heat are present. Using multiple sensors to monitor temperature of various parts of the system helps to build a model comprehensive thermal behavior model of the system and fine-tune the warm-up settings more accurately.

In an example, the camera-lens system is thermally modelled. In another example, the complete arrangement, i.e. system, frame, cover and cameras are thermally modelled. Based on the modelling, it is possible to determine the response of the system (transfer function) without having a priori knowledge of the system. In an example, main input for the model is a combination of signals from temperature sensors, camera imaging parameters, e.g. frame rate, system power consumption and image-based parameters, e.g. brightness of the image.

In an example, the temperature history is provided as a current temperature history. The temperature history can also be referred to as thermal history.

In an example, the thermal state of the system based temperature history is used to predict the time that remains before the system can be used for normal operation. Preferably, that information can be provided as feedback, e.g. as countdown timer, progress bar or the like.

In another option, a modified parameter set is provided after a start-up phase. For example, a system is operated at a significantly lower ambient temperature than during calibration. It is thus provided to run, i.e. operate, the camera at a slightly increased frame rate to compensate for the lower ambient temperature.

In an example, not further shown, the controller is configured to analyze at least two images from different points in time during the warm-up and to determine the current rate of displacement and to adjust the increased energy consumption mode accordingly.

In an example, shown as option in Fig. 2, a set of markers 82 is provided that can be tracked by the system. The markers 82 are attachable to at least one of the group of subjects, objects and devices.

The markers 82 are also referred to as fiducials.

The present solution avoids high costs of a navigation system. Further, clinical adoption of surgical navigation systems is also supported because the use of the navigation technology is facilitated and also fits better in the clinical routines due to its accelerated warm-up. Such medical, e.g. surgical navigation system works quick and easy with simplified procedures and is thus less complicating the workflow. The navigation systems are also suitable for being moved between operating rooms. The system is easy to setup, it boots-up quickly and it is ready for use within a few minutes.

Applying the above operation scheme will result in a particular energy consumption pattern, which may be detected by e.g. measuring the power consumption of the cameras in a given optical tracking system, for example using a USB power meter or measuring voltage and currents in the hardware.

In an example, a temperature measurement system is provided to measure the temperature of the cameras and/or other parts of the system.

In an example, a processor is provided to analyze at least two images and determine the current rate of displacement.

In an example, a timer to monitor the length of the warm-up period is provided.

In an example, a processor is provided that is capable of processing the transient warm-up period measurement information, configuring the cameras and receiving and processing image data from the cameras.

It is provided that the thermal stabilization of the optical tracking system is accelerated by configuring the cameras such that they to consume more power during warm-up than during regular operation. Further, once the desired thermal state is reached, the cameras are configured to provide the desired performance at the minimum power consumption possible.

Regarding the warm-up of an optical tracking system, roughly two stages can be distinguished. In stage 1, the temperature of the system increases steeply due to self-heating of the active components in the cameras. In stage 2, the system is approaching or has reached a thermal equilibrium. Stage 2 may be provided longer (several hours) than stage 1 (without supported heating-up: between half an hour and one hour; significantly shorter with supported (boosted) heating-up).

In an example, two fixed sets of image acquisition parameters are used: A first setting correlates with high-power demand of the cameras to heat-up the cameras. A second setting is associated with normal image acquisition by the cameras.

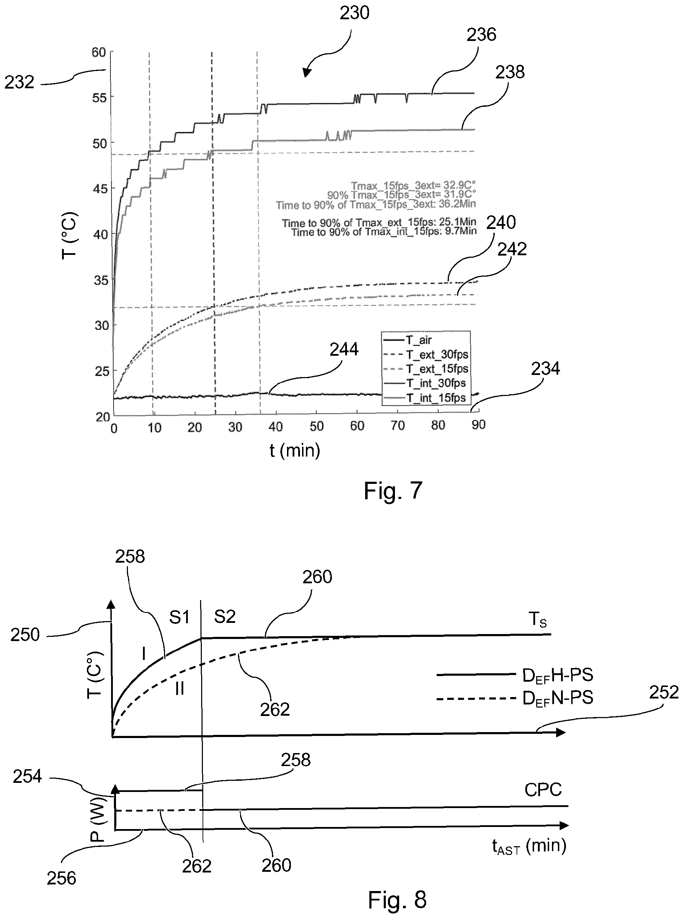

In an example, during the first stage of the warm-up of the cameras, the high-power settings are used to speed-up the warm-up of the cameras (Fig. 8, curve I) to a temperature level that would have been reached using the normal-power settings (Fig. 8, curve II). Once a preset temperature is reached, the system switches to settings needed for normal image acquisition by the cameras.

In an example, machine vision cameras can be controlled by configuring image acquisition parameters such as frame rate, exposure time, binning, bit depth, bit packing, area of interest, clocking frequency and the level of on-board image pre-processing, e.g. image compression, debayering. An example is shown in Fig. 7. The warm-up time of the camera imaging chip and camera housing is accelerated by a factor of about 3.5 and of about factor 1.4 respectively by using a higher frame-rate for the first part of the start-up.

In an example, similar to the previous example except that the image acquisition parameters for normal use of the cameras do not have a fixed value, once phase 2 is reached, the parameters are dynamically changed until the temperature is reached that is close to or equal to the temperature at the moment of calibration. The main advantage of this feature is that the system can mitigate - to a certain extent the effect of external conditions (e.g. ambient temperature, exposure to other heat sources, such as surgical lights).

In a further example, similar to the previous example except that the image acquisition parameters for normal usage during stage 2 are dynamically adjusted if external condition, e.g. ambient temperature, acquisition parameters change in order to maintain a system temperature close to the calibration temperature.

A further example is provided similar to the previous example, except that the system uses a model of the thermal behavior of the system to predict how to adjust image acquisition parameters such that a desired thermal stage/system temperature can be reached and maintained. Preferably, the system is self-learning and adjusting its model based on logged data of previous warm-up cycles and/or based on input from various sources, e.g. temperature sensors, imaging sensor.

In a further example, similar to the previous example, except that the camera is modelled and controlled according to minimum time control (or optimal control), the system reaches equilibrium with only two control actions (e.g. fastest transient possible). In such a case, the camera is configured to first consume as much power as possible; once a certain state is achieved, the camera is configured to consume as little power as possible until the desired state (steady state) is reached. Then the camera is configured as in steady state.

Fig. 4 shows steps of an example of a method 100 for operation of a camera-based tracking system. The method 100 comprises the following steps:
- In a first step 102, at least one operation signal is generated during a starting phase for the tracking system that comprises, at least during a starting phase of the tracking system, at least one calibration compensation instruction. The at least one calibration compensation instruction comprises instructions for at least one of the group of: i) actively adjusting a temperature of the camera-based tracking system, and ii) adapting a calibration-related camera output of the camera-based tracking system, which camera output is used for a tracking calculation.
- In a second step 104, the at least one operation signal is provided to the tracking system.
- In a third step 106, the camera-based tracking system is operated based on the operation signal comprising at least one calibration compensation instruction.

Actively adjusting the camera-based tracking system relates to adjusting the system in its geometrical setup. The active adjustment modifies the actual constructive setup. Adapting the camera output relates to modifying the image processing in terms of spatial registration of images provided by the cameras. The adaptation of the camera output modifies the virtual setup provided by the calibration.

In an example, indicated by hashed lines in Fig. 3, in an initial step 108, a signal for activating the camera-based tracking system is received.

In an example, as an option of the method, it is provided that generating the at least one operation signal for the tracking system comprises generating at least one control signal for temporary operating at least one of at least two cameras of the system in a heat-up mode to generate heat by the at least one of the at least two cameras. The at least one control signal is configured to operate the at least one of the at least two cameras in an increased energy consumption mode where a higher energy consumption by the camera takes place than in a regular tracking mode when the camera is operated to track markers. In the example, operating the camera-based tracking system comprises operating the at least one of the at least two cameras in the increased energy consumption mode and thereby heating up at least the camera due to a higher energy consumption as compared to the regular tracking mode.

The reduction of the divergence between the current condition of the camera-based tracking system and the predetermined condition of the system assumed for the calibration of the system is provided by the heating up of the at least one camera due to the higher energy consumption as compared to the regular tracking mode.

In an example, a method for operating a camera-based tracking system is provided. The method comprises the following steps:
- receiving a signal for activating a camera-based tracking system comprising at least two cameras;
- based on the activating signal, generating at least one operation signal for the tracking system that comprises at least one control signal for temporary operating at least one of at least two cameras in a heat-up mode to generate heat by the at least one of the at least two cameras;
   wherein the at least one control signal is configured to operate the at least one of the at least two cameras in an increased energy consumption mode where a higher energy consumption by the camera takes place than in a regular tracking mode when the camera is operated to track markers;
- providing the at least one operation signal to the tracking system; and
- operating the at least one of the at least two cameras in the increased energy consumption mode and thereby heating up at least the camera due to a higher energy consumption as compared to the regular tracking mode.

Thus, a speeding up of the warm-up curve is provided to reach a temperature that at least essentially resembles calibration temperature such that errors due to temperature-dependencies in the optical system are minimal.

In an example of the method, the at least one camera is operated in the increased energy consumption mode until a predetermined thermal condition is reached.

In an option, the increased energy consumption mode is maintained until at least one of the group of the at least one camera and the system has reached its normal operation temperature which is the basis for the system's calibration.

In another option, the increased energy consumption mode is provided beyond the point when the at least one camera has reached its normal operation temperature.

The normal operation temperature can also be referred to as the system's calibration temperature.

In an example, the at least one camera is operated in the increased energy consumption mode until a predetermined temperature of the camera is reached.

In another example, the at least one camera is operated in the increased energy consumption mode for a predetermined period of time.

In an example, the high-power consumption(s) is(are) maintained longer than needed to reach normal-operation temperature of the camera(s) such that the parts of the system that serve as heat-sink, e.g. camera holder or frame, do reach their target temperatures more quickly. After that, the system switches to lower-power consumption settings.

In an example, the part of the system that serves as heat-sink of the cameras is designed such that a thermal equilibrium of the system is reached as soon as possible. For example, it is provided to optimize thermal flow from the cameras to the frame such that the delay in frame warm-up, and associated suboptimal system performance, is minimized.

Preferably, the part of the system that serves as heat-sink of the cameras is designed such that a thermal equilibrium of the system is reached as soon as possible. An example can be to optimize thermal flow from the cameras to the frame such that the delay in frame warm-up (and associated suboptimal system performance) is minimized.

In an example of the method, after heating up the at least one camera by operating the at least one of the at least two cameras in the increased energy consumption mode, a normal operation signal is provided and the at least one camera is operated under normal conditions.

In an option, the settings for normal operation are modified based on the difference between the system temperature at time of calibration and the expected steady state temperature of the system under real-time circumstances.

In an example, the settings used for normal operation are not fixed, but tuned based on the difference between 1) the system temperature at time of calibration and 2) the expected steady-state temperature of the system under real-time circumstances. For example, if the system temperature at normal operation will be either higher or lower than the temperature at the time of calibration, the camera parameters are chosen such that the cameras consume either less or more power, thereby bringing the system in a state that is closer to the calibration state.

In an example, the camera settings for normal operation are optimized for minimal power consumption, while still providing adequate image data. This helps to the steady-state temperature of the optical system during normal operation, hence decreasing the temperature difference between cold-start conditions (cameras are at room temperature) and the temperature once the thermal equilibrium has been reached. The result is that not only less time is needed to reach normal operating temperature, but also that the system is subjected to less temperature variation (and associated decreased performance) during start-up.

In an example of the method, for generating the at least one operation signal for the tracking system, a model of the warm-up of the system and its dynamic thermal behavior is provided and taken into account for the at least one control signal. The at least one control signal comprises a signal sequence comprising a plurality of varying control sub-signals.

In an example of the method, it is provided to analyze at least two images from different points of time during the warm-up and determine the current rate of displacement and to adjust the increased energy consumption mode accordingly.

In an example of the method, a plurality of sensors is provided that monitor a thermal behavior of the system. The thermal behavior as monitored by the sensors is used for providing a more accurate model of the warm-up of the system. As an option, the model of the warm-up of the system is used in combination with a plurality of temperature readings from sensors during warm-up to dynamically adjust the calibration of the system. As a further option, a temperature history of the system is provided, and a remaining time is predicted before the system reaches a normal operation stage. Optionally, the remaining time is indicated.

In an example, provided as another option of the method, it is provided the steps of:
- providing a dynamic calibration model for the camera-based tracking system; wherein the dynamic calibration model comprises a plurality of calibration factors depending on the temperature related state of the system;
- providing a current temperature related state of the camera-based tracking system;
- determining a matching calibration factor from the plurality of calibration factors; and
- providing the matching calibration factor for application during operation of the camera-based tracking system.

Thus, a modified calibration is provided during starting phase that provides a matching temperature that at least essentially resembles the dynamic calibration temperature such that errors due to temperature-dependencies in the optical system are minimal.

In an example, the temperature related state comprises a temperature measurement from a sensor. For example, the sensor is provided within the system. In an option, the sensor is integrated, and provides temperature signals also for other system operating aspects. In an option, the sensor is provided for the calibration adjustment exclusively. According to an aspect, the temperature dependent behavior of the optical system is measured and modelled. Besides knowing the changing properties upfront (for example based on simulations or based on empiric data), additional temperature sensors can be provided to monitor the temperature of camera and supporting hardware in real-time.

Rather than using a static calibration that correlates with a steady-state, a dynamic calibration model is provided. In an example, the dynamic calibration model is dependent on time passing by since start-up. In an example, the dynamic calibration is dependent on the current temperature-condition, e.g. using temperature sensor readings. In an example, the dynamic calibration model is used considering time passing by since start-up and also the current temperature-condition.

In an example, a look-up table is provided upfront.

In one basic approach, only time since start-up is used to pick the right set of calibration parameters from the look-up table.

In another basic approach, a measurement from a temperature sensor is provided that is used to pick the right set of calibration parameters from the look-up table.

For example, an internal temperature sensor of one of the camera chips is used to pick the set of calibration parameters that most-probably give the best results.

In another, more advanced approach, the starting temperature of the camera in combination with the ambient temperature is used to estimate the warm-up curve and to thus provide the correct calibration factor.

In an example, the current temperature related state is updated, e.g. constantly or in changing intervals, to provide two or more updated calibration factors during warm,-up of the system.

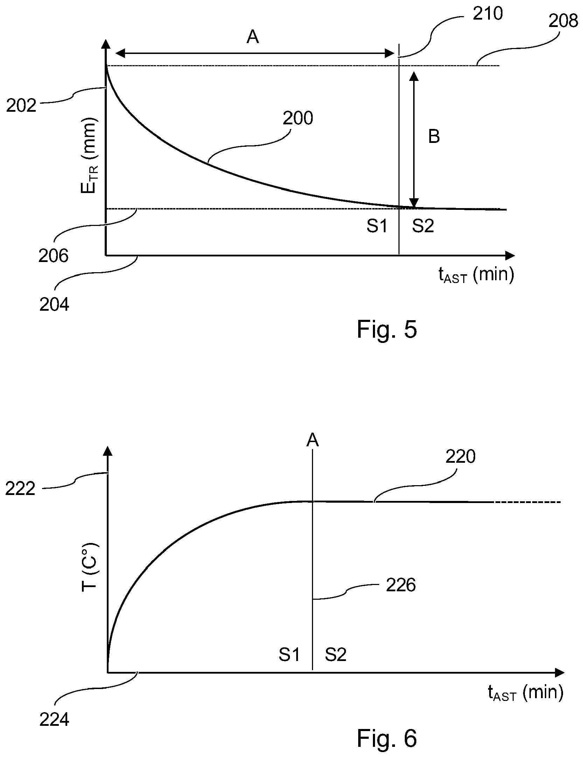

Fig. 5 shows a graph with a curve 200 indicating tracking error in relation to time after start. A vertical axis 202 indicates tracking error E_{TR} in millimeter; a horizontal axis 204 indicates time after start-up, t_{AST}, in minutes. During warm-up of an optical tracking system, the tracking error gradually becomes smaller until it reaches the system's baseline level 206 (intrinsic accuracy) once the temperature of the system has stabilized. Preferably, the time A needed to reach steady-state from maximum error level 208 is short to allow the user to use the system soon after start-up. The difference B in tracking errors associated with different temperature states of the system, e.g. cold start or operation temperature, is a measure for the system's robustness against temperature variation. A vertical line 210 separates a first stage S1, also referred to as stage 1, from a second stage S2, also referred to as stage 2.

Fig. 6 shows a graph with a curve 220 indicating temperature of the system in relation to time after start. A vertical axis 222 indicates temperature *T* in °C (degree Celsius); a horizontal axis 224 indicates the time after start-up, t_{AST}, in minutes. The graph can be divided in a warm-up phase, e.g. stage S1, and a steadier operation phase, e.g. stage S2. The separation is indicated with a vertical line 226.

Fig. 7 shows a graph 230 with different curves indicating system temperature over time for different operating modes. A vertical axis 232 indicates temperature *T* in °C; a horizontal axis 234 indicates time t (in min). With an optical tracking system comprising industry-standard machine vision cameras, the measured power consumption of the cameras can be increased by changing image acquisition parameters. In this particular example, the power consumption is increased from 2,6 W to 2,96 W by increasing the frame rate from 15 to 30 frames per second (fps). Warm-up of the camera imaging chip and camera housing was accelerated by a factor 3.5 and factor 1.4, respectively, by boosting warm-up using a higher frame rate. A first curve 236 indicates the camera internal temperature for 30 fps, and a second curve 238 indicates the camera internal temperature for 15 fps. A third curve 240 indicates the external temperature, i.e. the temperature outside the camera, for 30 fps; and a fourth curve 242 indicates the external temperature, i.e. the temperature outside the camera, for 15 fps. A further curve 244 indicates the air temperature. As can be seen, the increase of the frame rate leads to a significant increase in temperature during the initial phase when temperature is steeply rising to then flatten towards a steady state temperature.

Fig. 8 shows a graph indicating an example of a warm-up pattern with system temperature and camera power consumption over time. In the upper graph, a vertical axis 250 indicates the temperature T in °C; a horizontal axis 252 indicates the time after start-up, t_{AST}, in minutes. In the lower graph, a vertical axis 254 indicates the power consumption P of the camera in W; and the time after start-up, t_{AST}, in minutes is again indicated on a horizontal axis 256. During stage S1, default high-power demanding camera settings, D_{EF}H-PS, indicated with a first curve 258 are used to accelerate the warm-up of the system. Once stage S2 is reached, the system switches to default settings for normal use of the cameras, indicated with a steady line portion 260 of the temperature curve Tₛ. For comparison, a further curve 262 indicates default normal power settings, D_{EF}N-PS. In the upper graph, a horizontal line 284 indicates a calibration temperature, T_{C}. As can be seen, the application of the high-power demanding camera settings leads to an increase in temperature resulting in an earlier time of achieving the operational temperature.

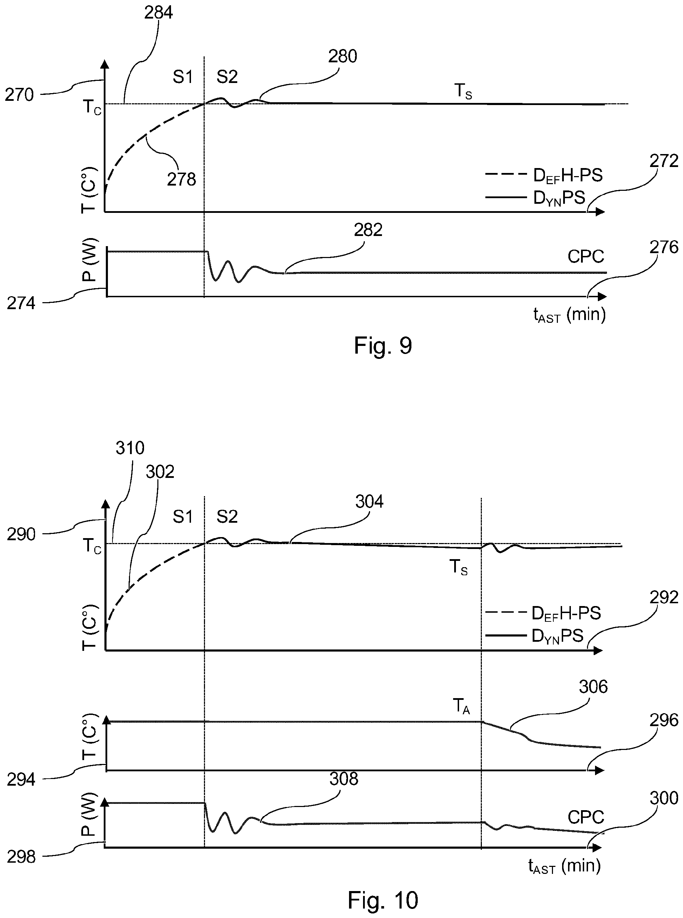

Fig. 9 shows a graph indicating another example of a warm-up pattern with system temperature and camera power consumption over time. In the upper graph, a vertical axis 270 indicates the temperature T in °C; and a horizontal axis 272 indicates the time *t* in minutes. In the lower graph, a vertical axis 274 indicates the power consumption P of the camera in W; and a horizontal axis 276 again indicates the time t in minutes. At the start of stage S2, image acquisition parameters and associated camera power consumption are dynamically adjusted until it reaches the calibration temperature. A first part 278 of a curve indicate the application of default high-power settings, D_{EF}H-PS, a second part 280 of the curve indicate the application of dynamic power settings, D_{YN}PS. A curve 282 in the lower graph indicates the varying camera power consumption, CPC. In the upper graph, again, a horizontal line 310 indicates a calibration temperature T_{C}. As can be seen, a dynamic adaptation of the power consumption is provided in a transition period at the boundary of the first stage, S1, and the second stage, S2.

Fig. 10 shows a graph indicating a further example of a warm-up pattern with system temperature and camera power consumption over time. In the upper graph, a vertical axis 290 indicates the system temperature T_{S} in °C; a horizontal axis 292 indicates the time after start-up t_{AST}, in minutes. In the middle graph, a vertical axis 294 indicates the ambient temperature T_{A} in °C; a horizontal axis 296 indicates again the time after start-up, t_{AST}, in minutes. In the lower graph, a vertical axis 298 indicates the power consumption P of the camera in W; a horizontal axis 300 indicates the time after start-up t_{AST}, in minutes. During stage S2, the image acquisition parameters (and associated camera power consumption) are dynamically adjusted to mitigate the effect of variations in external conditions, e.g. ambient temperature, such that a temperature close to the system temperature at the time of calibration is maintained. A first part 302 indicates the application of default high-power settings, D_{EF}H-PS; a second part 304 of the curve indicate the application of dynamic power settings, D_{YN}PS. A curve 306 in the middle graph indicates a drop in ambient temperature. A curve 308 in the lower graph indicates the varying camera power consumption, CPC. As can be seen, even after the initial heat-up, further adjustments of the system temperature may be addressed in view of calibration during the operation. As an example, a change in the ambient temperature is indicated. As a reaction, the consumer power of the camera is adjusted accordingly.

The term "subject" may also be referred to as individual. The "subject" may further also be referred to as patient, although it is noted that this term does not indicate whether any illness or disease is actually present with the subject.

In an example, a computer program or program element for controlling an apparatus according to one of the examples above is provided, which program or program element, when being executed by a processing unit, is adapted to perform the method steps of one of the method examples above.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it, which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A control device (10) for operation of a camera-based tracking system, the control device comprising:
- a controller (12); and
- a control signal output (14);
wherein the controller is configured to generate at least one operation signal for the tracking system that comprises, at least during a starting phase of the tracking system, at least one calibration compensation instruction;
wherein the at least one calibration compensation instruction comprises instructions for at least one of the group of: i) actively adjusting a temperature of the camera-based tracking system, and ii) adapting a calibration-related camera output of the camera-based tracking system, which camera output is used for a tracking calculation; and
wherein the control signal output is configured to provide the at least one operation signal to the tracking system.

2. Control device according to claim 1, wherein the calibration compensation instruction comprises at least one control signal for temporary operating the camera-based tracking system in an increased heat generating mode.

3. Control device according to claim 2, wherein, for temporary operating the camera-based tracking system in the increased heat generating mode, the calibration compensation instruction comprises at least one control signal for temporary operating at least one of the at least two cameras in a heat-up mode to generate heat by the at least one of the at least two cameras; wherein the at least one control signal is configured to operate the at least one of the at least two cameras in an increased energy consumption mode where a higher energy consumption by the camera takes place than in a regular tracking mode when the camera is operated for tracking; and
wherein the control signal output is configured to provide the at least one operation signal to the tracking system in order to operate the at least one of the at least two cameras in the increased energy consumption mode and to thereby heat up at least the camera due to a higher energy consumption as compared to the regular tracking mode.

4. Control device according to claim 2, wherein, for the increased heat generating mode, the calibration compensation instruction comprises at least one control signal for temporary operating a heating element of the camera-based tracking system.

5. Control device according to claim 2, 3 or 4, wherein the controller is configured to take measured external conditions into account for the control signal when generating the operation signal, the external conditions comprising at least one of the group of:
- ambient temperature;
- illumination conditions; and
- indoor air currents.

6. Control device according to one of the claims 2 to 5, wherein, for generating the at least one operation signal for the tracking system, the controller is configured to provide a model of the warm-up of the system and its dynamic thermal behavior and to take these into account for the at least one control signal; and
wherein the at least one control signal comprises a signal sequence comprising a plurality of varying control sub-signals.

7. Control device according to one of the preceding claims, wherein the controller is configured to provide a dynamic calibration model for the camera-based tracking system, the dynamic calibration model comprising a plurality of calibration factors depending on a temperature related state of the system; and
wherein, for a current temperature related state of the camera-based tracking system, the controller is configured to determine a matching calibration factor from the plurality of calibration factors; and
wherein the control signal output is configured to provide the matching calibration factor for application during operation of the camera-based tracking system.

8. A camera-based tracking system (50), comprising:
- a plurality of at least two cameras (52); and
- a control device (10) according to one of the preceding claims;
wherein the at least one operation signal is provided for operating at least one of the at least two cameras; and
wherein the camera-based tracking system is a camera-based medical tracking system for use in a hospital or other healthcare related setting.

9. System according to claim 8, wherein the increased energy consumption mode comprises an image acquisition with an energy consumption that is at least 50% higher than an energy consumption during a normal tracking mode of the system; and
wherein the increased energy consumption mode comprises an image acquisition with at least one of the group of the following being at least 50% increase compared to a normal tracking mode of the system:
- frame rate;
- exposure time;
- bit depths;
- bit packing;
- area of interest;
- clocking frequency;
- color formats;
- image compression; and
- on-board image pre-processing.

10. System according to claim 8 or 9, wherein a plurality of sensors (84) is provided that monitor a thermal behavior of the system;
wherein the thermal behavior as monitored by the sensors is used for providing a more accurate model of a warm-up of the system;
wherein the model of the warm-up of the system is used in combination with a plurality of temperature readings from sensors during warm-up to dynamically adjust the calibration of the system; and
wherein a temperature history of the system is provided, and a remaining time is predicted before the system reaches a normal operation stage, and the remaining time is indicated.

11. System according to one of the claims 8 to 10, wherein the controller is configured to analyze at least two images from different points in time during the warm-up and to determine the current rate of displacement and to adjust the increased energy consumption mode accordingly.

12. System according to one of the claims 8 to 11, wherein a set of markers (82) is provided that can be tracked by the system; and
wherein the markers are attachable to at least one of the group of subjects, objects and devices.

13. A method (100) for operation of a camera-based tracking system, comprising the following steps:
- generating (102) at least one operation signal for the tracking system that comprises, at least during a starting phase of the tracking system, at least one calibration compensation instruction;
wherein the at least one calibration compensation instruction comprises instructions for at least one of the group of: i) actively adjusting a temperature of the camera-based tracking system, and ii) adapting a calibration-related camera output of the camera-based tracking system, which camera output is used for a tracking calculation;
- providing (108) the at least one operation signal to the tracking system; and
- operating (110) the camera-based tracking system based on the operation signal comprising at least one calibration compensation instruction.

14. A computer program enabling a processor to carry out the method of claim 13.

15. A computer readable medium having stored the program element of claim 14.
